# EUROPEAN PATENT APPLICATION

(11) **EP 4 744 586 A1**
(43) Date of publication of application: **20.05.2026**
(21) Application number: 25215747.4
(22) Date of filing: 13.11.2025
(51) Int. Cl.: A61B 5/103, A61B 5/00

(54) **PERFORMING MEASUREMENTS FOR IDENTIFYING A PILLOW**

(30) Priority: 15.11.2024 NL 2039090
(71) Applicant: Beter Bed B.V., 5405 AR Uden (NL)
(72) Inventor: Van Seumeren, Cyril Simon, 5405 AR Uden (NL); Burbach, Mark, 5405 AR Uden (NL); Van der Loo, Bram Willem, 5405 AR Uden (NL)
(74) Representative: De Vries & Metman

(57) **Abstract**

A system (20) for performing measurements in relation to a person comprises a head measuring component (33) in the form of a pillow and a body measuring component (31) in the form of a bed. The measurements enable one or more suitable pillows to be identified for the person. The head measuring component comprises at least one head sensor (51-54). The body measuring component comprises at least one third sensor (43,44) at at least one position which corresponds to a top part of the person's body and at least one fourth sensor (41,42) at at least one position which corresponds to a bottom part of the person's body. The sensors are configured to perform measurements indicative of a respective force applied to the respective sensor.

## Description

### FIELD OF THE INVENTION

The invention relates to a system for performing measurements in relation to a person, the measurements enabling one or more suitable pillows to be identified for the person.

The invention further relates to a method of identifying one or more suitable pillows for a person based on measurements.

The invention also relates to a computer program product enabling a computer system to perform such a method.

### BACKGROUND OF THE INVENTION

Finding the best pillow for individual sleep needs is a complex challenge, stemming from the unique requirements each person has based on their body type, sleep position, mattress firmness, and personal comfort preferences. A poor pillow choice can lead to sleep disruption, neck and shoulder pain, and even exacerbate certain health issues, such as snoring or sleep apnea.

Devices have been invented to help identify one or more suitable pillows for a person. For example, KR101626451 B1 describes a head pressure measuring apparatus that includes a bed with a head positioning part and a body positioning part. The device has sensors in the head positioning part to measure the pressure applied by the user's head. This allows the device to accurately measure the weight of the user's head. The device can also have multiple sensors arranged in a grid pattern to map the pressure distribution on the head. This pressure map can be used to recommend a suitable pillow for the user.

Additionally, the device includes a size measurement module to measure the width of the user's head, and a length measurement module to measure the length of the head and neck. This allows the device to provide comprehensive measurements for selecting a customized pillow. However, with these measurement results, it is still not trivial to identify one or more suitable pillows.

### SUMMARY OF THE INVENTION

It is advantageous to provide a system and method, which allow one or more suitable pillows to be identified for a person in a trustworthy and easy manner.

In a first aspect, a system for performing measurements in relation to a person, the measurements enabling one or more suitable pillows to be identified for the person, comprises a head measuring component in the form of a pillow, the head measuring component comprising at least one head sensor, the at least one head sensor being configured to perform first measurements indicative of a respective force applied to the respective sensor, and a body measuring component in the form of a bed, the body measuring component comprising at least one third sensor at at least one position of the bed which corresponds to a top part of the person's body and at least one fourth sensor at at least one position of the bed which corresponds to a bottom part of the person's body, the at least one third sensor and the at least one fourth sensor being configured to perform second measurements indicative of a respective force applied to the respective sensor.

By not only having one or more sensors of a head measuring component perform measurements indicative of a respective force but by also having top and bottom sensors of a body measuring component perform measurements indicative of a respective force, the obtained measurement results depend (to a larger degree) on the person's body type and this allows a suitable pillow to be identified for the person in a trustworthy and easy manner. It is not just the weight of the person that is measured, but the weight distribution of the person. The inventors have recognized that there is a relation between this weight distribution and the pillows which are most suitable for a person.

A suitable pillow provides support for the neck and head, ensuring that the cervical vertebrae are aligned in a straight line with the head and thoracic vertebrae. The support provided by a pillow is determined by its height, surface area, shape, and filling material.

The body measuring component may comprise at least two third sensors at first and second positions of the bed which correspond to a top part of the person's body and at least two fourth sensors at third and fourth positions of the bed which correspond to a bottom part of the person's body, the first and third positions of the bed corresponding to a left part of the person's body and the second and fourth positions of the bed corresponding to a right part of the person's body.

The at least one head sensor may comprise at least one first sensor at at least one position of the pillow which corresponds to a top part of the person's head and at least one second sensor at at least one position of the pillow which corresponds to a bottom part of the person's head.

The at least one head sensor may comprise at least two first sensors at first and second positions of the pillow which correspond to a top part of the person's head and at least two second sensors at third and fourth positions of the pillow which correspond to a bottom part of the person's head, the first and third positions of the pillow corresponding to a left part of the person's head and the second and fourth positions of the pillow corresponding to a right part of the person's head.

The bed may comprise one or more ground supports which allow the bed to stand on the ground and the at least one third sensor and the at least one fourth sensor are mounted below the one or more ground supports.

The system may further comprise at least one processor configured to obtain first measurement results and second measurement results, the first measurement results comprising the first measurements or being derived from the first measurements, the second measurement results comprising the second measurements or being derived from the second measurements, determine one or more target properties for a target pillow based on the first measurement results and the second measurement results, identify the one or more suitable pillows from a plurality of pillows by comparing the one or more target properties with a plurality of measured properties per pillow of the plurality of pillows.

The at least one processor may be configured to determine the one or more target properties for the target pillow by determining a target support height range or value and a target counter pressure range or value based on the first measurement results and the second measurement results, and identify the one or more suitable pillows from the plurality of pillows by comparing the target support height range or value and the target counter pressure range or value with the plurality of measured properties per pillow, the plurality of measured properties per pillow comprising a plurality of pairs of pillow characteristics per pillow, each of the pairs comprising a measured counter pressure and a corresponding value indicative of a support height.

The plurality of measured properties per pillow may have been obtained by automatically controlling an artificial head to achieve the different support heights of a respective pillow of the plurality of pillows and measuring the corresponding counter pressure at each of the different support heights.

A first subset of the plurality of measured properties per pillow may have been obtained by automatically controlling the artificial head with a back of the artificial head and a second subset of the plurality of measured properties per pillow may have been obtained by automatically controlling the artificial head with a side of the artificial head.

The system may further comprise a device for automatically controlling the artificial head to achieve the different support heights and measuring the corresponding counter pressure at each of the different support heights.

The system may further comprise a user terminal configured to provide questions to a user and receive answers to the questions from the user, and wherein the at least one processor may be further configured to determine the one or more target properties for the target pillow further based on at least a subset of the answers and/or to select the plurality of pillows from a collection of pillows based on at least a subset of the answers.

The questions may ask the user about one or more of: a firmness of a mattrass of the user, a favorite sleeping position of the user, pain experienced by the user when lying in bed, comfortability of the user in terms of temperature when lying in bed, and materials to be excluded.

One of the questions may ask the user about the firmness of the mattrass of the user and the system may further comprise an adjustable bed base and a controller for adjusting the adjustable bed base based on the user's answer to the question about the firmness of the mattrass of the user.

The second measurement results may comprise at least one average top measurement and at least one average bottom measurement, the average top measurement being an average of measurements performed by the least two third sensors at the first and second positions of the bed and the average bottom measurement being an average of measurements performed by the least two fourth sensors at the third and fourth positions of the bed. The at least one processor may be configured to determine the one or more target properties for the target pillow based on the first measurement results, the average top measurement, and the average bottom measurement.

In a second aspect, a method of identifying one or more suitable pillows for a person based on measurements comprises obtaining first measurement results and second measurement results, the first measurement results comprising first measurements or being derived from the first measurements, the second measurement results comprising second measurements or being derived from the second measurements, the first measurements being indicative of a respective force applied to a respective sensor of at least one head sensor, the second measurements being indicative of a respective force applied to a respective sensor of at least one third sensor and at least one fourth sensor, the at least one third sensor being positioned at at least one position of a bed which corresponds to a top part of the person's body and the at least one fourth sensor being positioned at at least one position of the bed which corresponds to a bottom part of the person's body.

The method further comprises determining one or more target properties for a target pillow based on the first measurement results and the second measurement results and identifying the one or more suitable pillows from a plurality of pillows by comparing the one or more target properties with a plurality of measured properties per pillow of the plurality of pillows. The method may be performed by software running on a programmable device. This software may be provided as a computer program product.

Moreover, a computer program for carrying out the methods described herein, as well as a non-transitory computer readable storage-medium storing the computer program are provided. A computer program may, for example, be downloaded by or uploaded to an existing device or be stored upon manufacturing of these systems.

In another aspect, a non-transitory computer-readable storage medium stores a software code portion, the software code portion, when executed or processed by a computer, being configured to perform the method described above.

As will be appreciated by one skilled in the art, aspects of the present invention may take the form of a device, a method or a computer program product. Accordingly, aspects of the present invention may take the form of an entirely hardware implementation, an entirely software implementation (including firmware, resident software, micro-code, etc.) or an implementation combining software and hardware aspects that may all generally be referred to herein as a "circuit", "module" or "system." Functions described in this disclosure may be implemented as an algorithm executed by a processor/microprocessor of a computer. Furthermore, aspects of the present invention may take the form of a computer program product in one or more computer readable medium(s) having computer readable program code stored thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples of a computer readable storage medium may include, but are not limited to, the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present invention, a computer readable storage medium may be any tangible medium that can contain, or store, a program for use by or in connection with an instruction execution system, apparatus, or device.

A computer readable signal medium may include a propagated data signal with computer readable program code included therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code on a computer readable medium may be transmitted using any appropriate medium, including but not limited to wireless, wireline, optical fiber, cable, RF, etc., or any suitable combination of the foregoing. Computer program code for carrying out operations for aspects of the present invention may be written in any combination of one or more programming languages, including an object oriented programming language such as Java(TM), Swift, Dart, Python, Go, C++ or the like and conventional procedural programming languages, such as the "C" programming language or similar programming languages. The program code may execute entirely on the user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems), and computer program products according to implementations of the present invention. It will be understood that each block of the flowchart illustrations and/or block diagrams, and combinations of blocks in the flowchart illustrations and/or block diagrams, can be implemented by computer program instructions. These computer program instructions may be provided to a processor, in particular a microprocessor or a central processing unit (CPU), of a general purpose computer, special purpose computer, or other programmable data processing apparatus to produce a machine, such that the instructions, which execute via the processor of the computer, other programmable data processing apparatus, or other devices create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the figures illustrate the architecture, functionality, and operation of possible implementations of devices, methods and computer program products according to various implementations of the present invention. In this regard, each block in the flowchart or block diagrams may represent a module, segment, or portion of code, which comprises one or more executable instructions for implementing the specified logical function(s). It should also be noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

### BRIEF DESCRIPTION OF THE DRAWINGS

These and other aspects of the invention are apparent from and will be further elucidated, by way of example, with reference to the drawings, in which:
Fig. 1 shows implementations of head and body measuring components;
Fig. 2 shows a close-up view of a part of the body measuring component of Fig. 1;
Fig. 3 shows a block diagram of the head measuring component of Fig. 1;
Fig. 4 is a block diagram of an implementation of the system;
Fig. 5 shows a close-up view of the pillow indexer of Fig. 4;
Fig. 6 is a flow chart of a first implementation of the method;
Fig. 7 is a flow chart of a second implementation of the method;
Fig. 8 is a flow chart of a third implementation of the method;
Figs. 9-11 show example measurement properties for multiple pillows; and
Fig. 12 is a block diagram of an exemplary data processing system for performing the methods of the invention.

Corresponding elements in the drawings are denoted by the same reference numeral.

### DETAILED DESCRIPTION

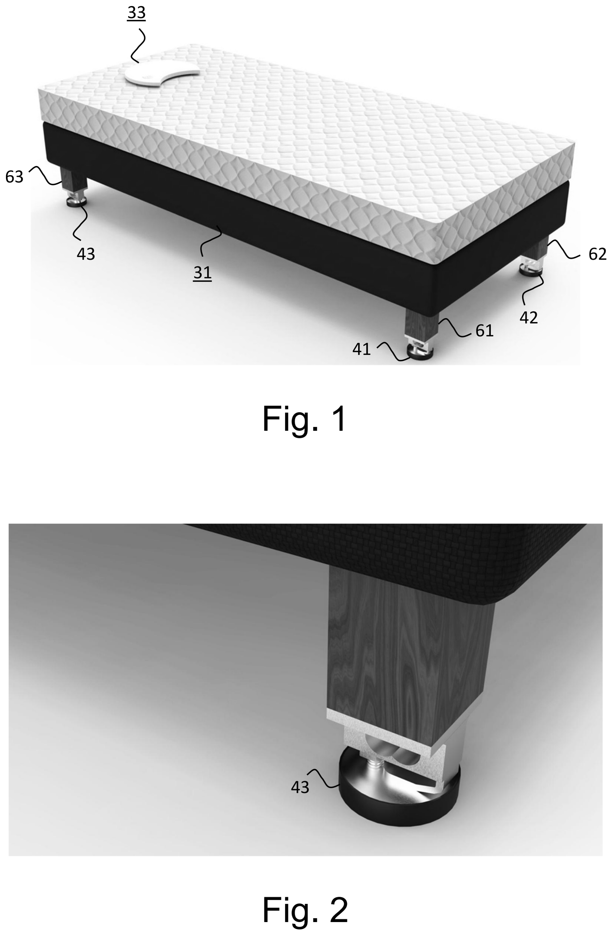

Fig. 1 shows implementations of a head measuring component 33 in the form of a pillow and a body measuring component 31 in the form of a bed. The head and body measuring components 33 and 31 are jointly referred to as "customer indexer" in this specification. The customer indexer may be used in stores to gather data on customer's body type and sleep preferences. With matching software, the best-suited pillow for the customer's personal sleep needs may be identified.

The head measuring component 33 comprises at least one head sensor. In the implementation of Fig. 1, the at least one head sensor comprises at least one first sensor at at least one position of the pillow which corresponds to a top part of the person's head and at least one second sensor at at least one position of the pillow which corresponds to a bottom part of the person's head. The at least one first sensor and the at least one second sensor are configured to perform first measurements indicative of a respective force applied to the respective sensor. In an alternative implementation, only one head sensor is used.

For example, the head measuring component 33 may be a head weight scale which measures head weight with a fixed height of 30 millimeters to assess how much weight is applied specifically in the head area. This value helps in determining the level of support required by the pillow. If multiple head sensors are used, an average may be determined from the measurements of all head sensors, averages may be determined from measurements performed by certain sets of head sensors, or the measurements of all head sensors may be processed individually.

The body measuring component 31 comprises at least one third sensor at at least one position of the bed which corresponds to a top part of the person's body and at least one fourth sensor at at least one position of the bed which corresponds to a bottom part of the person's body. The at least one third sensor and the at least one fourth sensor are configured to perform second measurements indicative of a respective force applied to the respective sensor.

From these measurements, head weight, shoulder area weight, and overall weight distribution across the top and bottom of the bed may be determined. The sensors may be weight sensors, for example. Weight measurements (e.g., in kilograms) may be converted to values specifying force (e.g., in Newton). Alternatively, the sensors may measure force directly, for example. The sensors may be (flat) load cells, for example.

The body measuring component may comprise two or more third sensors at first and second positions of the bed which correspond to a top part of the person's body and two or more fourth sensors at third and fourth positions of the bed which correspond to a bottom part of the person's body. The first and third positions of the bed correspond to a left part of the person's body and the second and fourth positions of the bed correspond to a right part of the person's body.

In the implementation of Fig. 1, sensor 43 is located at the first position (top-left), sensor 44 is located at the second position (top-right; not visible in Fig. 1), sensor 41 is positioned at the third position (bottom-left), and sensor 42 is positioned at the fourth position (bottom-right). These sensors make it possible to calculate the weight distribution ratio across the upper and lower body, providing insights into whether a person's upper body requires additional support. Fig. 2 shows a close-up view of sensor 43.

The bed comprises one or more ground supports which allow the bed to stand on the ground and the at least one third sensor and the at least one fourth sensor are mounted below the one or more ground supports. In the implementation of Fig. 1, the bed 31 comprises legs 61-64 as ground supports (leg 64 is not visible in Fig. 1). In an alternative implementation, the bed frame is supported by side panels or a solid base, rather than individual legs.

In the implementation of Fig. 1, one load cell may be positioned on each corner of the bed frame leg. The bed may have a size of 2000 x 900 millimeters, for example. The head measuring component 33 may be a weight scale with a fixed position and a height of 30 millimeters and may be positioned 50 millimeters from the top of the bed, for example.

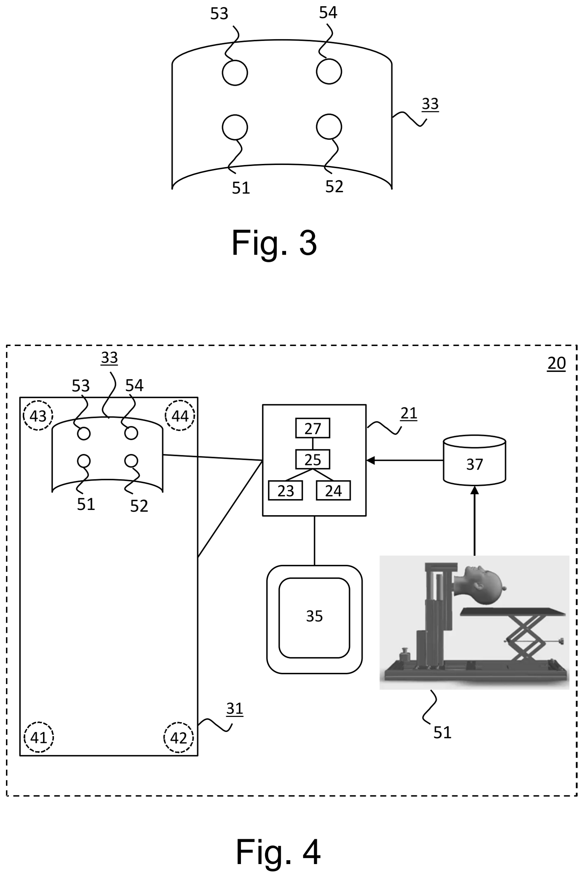

Fig. 3 shows a block diagram of the head measuring component 33 of Fig. 1. The head measuring component 33 comprises at least two first sensors at first and second positions of the pillow which correspond to a top part of the person's head and at least two second sensors at third and fourth positions of the pillow which correspond to a bottom part of the person's head, The first and third positions of the pillow corresponding to a left part of the person's head and the second and fourth positions of the pillow corresponding to a right part of the person's head.

In the implementation of Fig. 3, sensor 53 is located at the first position (top-left), sensor 54 is located at the second position (top-right), sensor 51 is positioned at the third position (bottom-left), and sensor 52 is positioned at the fourth position (bottom-right).

An implementation of the system for performing measurements in relation to a person is shown in Fig. 4. The measurements enable one or more suitable pillows to be identified for the person. In the implementation of Fig. 4, the system 20 comprises not only the customer indexer, i.e., the body and head measuring components 31 and 33, but also a control device 21 which comprises a processor 25, a receiver 23, a transmitter 24, and memory 27.

The processor 25 is configured to obtain first measurement results and second measurement results. The first measurement results comprise the first measurements or are derived from the first measurements. The second measurement results comprise the second measurements or are derived from the second measurements.

The second measurement results may comprise an average top measurement and an average bottom measurement. The average top measurement is an average of measurements performed by the least two third sensors at the first and second positions of the bed and the average bottom measurement is an average of measurements performed by the least two fourth sensors at the third and fourth positions of the bed. These averages may be calculated by the body measurement component 31 or by the processor 25, for example. Alternatively, the second measurement results may comprise the measurements performed by the four sensors 41-44 (i.e., no averages are determined), for example.

The first measurement results may also comprise an average top measurement and an average bottom measurement. The average top measurement is an average of measurements performed by the least two first sensors at the first and second positions of the pillow and the average bottom measurement is an average of measurements performed by the least two second sensors at the third and fourth positions of the pillow. These averages may be calculated by the head measurement component 33 or by the processor 25, for example. Alternatively, the second measurement results may comprise the measurement performed by the four sensors 51-54 (i.e., no averages are determined), for example.

The processor 25 is further configured to determine one or more target properties for a target pillow based on the first measurement results and the second measurement results. If the second measurement results comprise an average top measurement and an average bottom measurement, the processor 25 may be configured to determine the one or more target properties for the target pillow based on the first measurement results, this average top measurement, and this average bottom measurement.

If the first measurement results comprise an average top measurement and an average bottom measurement, the processor 25 may be configured to determine the one or more target properties for the target pillow based on the second measurement results, this average top measurement, and this average bottom measurement. The processor 25 may be configured to determine the one or more target properties for the target pillow by determining a target support height range or value and a target counter pressure range or value based on the first measurement results and the second measurement results.

The processor 25 is further configured to identify the one or more suitable pillows from a plurality of pillows by comparing the one or more target properties with a plurality of measured properties per pillow of the plurality of pillows. Thus, the control device 21 is able to automatically identify the one or more suitable pillows and measurements do not need to be interpreted manually.

For example, a target pillow support may be determined based on a person's sleeping position, head weight, shoulder area weight, and overall weight distribution across the top and bottom of the bed, and one or more suitable pillows may be identified which have this target pillow support. This control device 21 can thus provide individualized pillow recommendations based on the user's unique body profile and sleeping position.

In the implementation of Fig. 4, the plurality of measured properties per pillow are received from database 37. Database 37 comprises characteristics for each available pillow, such as pillow height and support profile. At least some of the characteristics are measured properties. The data from database 37 may be dynamically adjusted to fit different head sizes (or head weights) and body weights. The body weight of the person-under-test may be obtained from the body measuring component 31.

If the data is dynamically adjusted to fit different head sizes, the head size of the person-under-test may be estimated based on the body weight and/or head weight, for example. A default head diameter of 52 cm may be used. The system may be able to adjust for larger or smaller heads.

A benefit of having separate measurements from the at least one third sensor and the at least one fourth sensor (e.g., top and bottom load cells) of the body measuring component 31 is that they can be compared:
- Balanced Weight Distribution: if the weight distribution across the sensors (e.g., load cells) is balanced, standard support heights based on body weight may be recommended.
- Upper Body Weight Concentration: If the at least one top sensor measures a higher weight than the at least one bottom sensor, indicating a larger upper body mass (e.g., broader shoulders or more torso weight), the system may recommend a support height that deviates from the default support height to provide additional head support. This is important for maintaining spinal alignment and comfort, especially for back and side sleepers.

A default support height may be determined, for example, based on body weight and sleep position. For example, a default support height of 7 centimeters has been determined for an average person of 59 kilograms with balanced weight distribution. This has been determined based on empirical research and biomechanical modelling. The 7 centimeters support height recommendation is validated by research showing minimized cranio-cervical pressure, maintained spine alignment, and improved comfort for balanced individuals. The findings support optimal cervical support and alignment.

### Supporting Case Studies

1. Effect of Pillow Height on Cranio-Cervical Pressure
   Study: Ren et al. (2016), PeerJ
   Summary: Investigated the impact of support height on cranio-cervical pressure and cervical alignment using pressure-sensing and modeling.
   Relevance: Found that lower support heights (6-8 cm) minimized neck strain for back sleepers.
2. The Effect of Different Support Heights on Cervical Alignment
   Study: Leilnahari et al. (2011), BioMedical Engineering OnLine
   Summary: Examined support heights' effect on spine alignment, finding 6-9 cm ideal for various positions.
   Relevance: Supports the 7 cm recommendation for balanced spine alignment.
3. Ergonomics in Bed Design: Effect of Spinal Alignment on Sleep Quality
   Study: Verhaert et al. (2011), Ergonomics
   Summary: Studied support height and mattress firmness effects on alignment and quality.
   Relevance: 7 cm height is optimal for back sleepers, ensuring comfort and support.
4. Comparison of Cervical Pillow Types for Comfort
   Study: Persson & Moritz (1998), Journal of Manipulative and Physiological Therapeutics
   Summary: Compared different pillows, finding 6-8 cm support height is best for back sleepers.
   Relevance: Supports using 7 cm support height for balanced neck support.

### Calculations for Optimal Support Height

1. Cranio-Cervical Pressure Calculations
   - Force exerted by a 4.5 kg head (balanced 59 kg person) = 44.1 N
   - Pressure with 120 cm² contact area = 0.37 kPa
2. Support Height Determination
   Based on biomechanics, 7 cm height aligns with cranio-cervical and spinal alignment data.
3. Scaling for Different Body Types
   For larger body types, adjustments are calculated proportionally.
4. Mattress Firmness Impact
   Firm mattresses align with 7 cm height; softer may require +1 cm.

Deviations from the default support height may then be determined based on the separate top and bottom measurements of the body measuring component 31. An expected weight may be determined based on the body weight. This may depend on the country in which the system is used. For example, in the Netherlands, a head weight of approximately 7.6% of the total body weight may be used. If less weight is measured by the head measuring component 33 than expected and a higher weight is measured by the upper sensor(s) of the body measuring component 31 than expected, the system may determine that target support height should be higher than the default support height to provide the needed head support. This real-time adjustment ensures the support height match the user's body structure, promoting optimal alignment and comfort.

Examples are provided below:
**59 kg Person**
   ▪ Expected Head Weight on Scale: ~4.72 kg (8% of body weight).
   ▪Target Support Height:
      ∘ Back Sleeper: 6-8 cm (standard). If the top load cells show a higher weight concentration, the system increases the default support height to enhance head support.
      ∘ Side Sleeper: 8-10 cm, with potential increases for upper body support if needed.
      ∘ Stomach Sleeper: 3-5 cm to minimize neck strain.
**72 kg Person**
   ▪ Expected Head Weight on Scale: ~5.76 kg (8% of body weight).
   ▪ Target Support Height:
      ∘ Back Sleeper: 8-10 cm. For individuals with greater upper body weight, the system recommends increasing the default support height to support the head.
      ∘ Side Sleeper: 10-12 cm, with adjustments based on upper-to-lower body ratio.
      ∘ Stomach Sleeper: 4-6 cm.
**80 kg Person**
   ▪ Expected Head Weight on Scale: ~6.4 kg.
   ▪ Target Support Height:
      ∘ Back Sleeper: 9-11 cm, adjusted upwards if upper body weight is concentrated on top load cells.
      ∘ Side Sleeper: 11-13 cm, particularly for those with broader shoulders.
      ∘ Stomach Sleeper: 5-7 cm.

**Table 1: Example target support heights**

| **Body Weight** | **Sleeping Position** | **Target support height** |
|---|---|---|
| 59 kg | Back Sleeper | 6-8 cm |
| 59 kg | Side Sleeper | 8-10 cm |
| 59 kg | Stomach Sleeper | 3-5 cm |
| 72 kg | Back Sleeper | 8-10 cm |
| 72 kg | Side Sleeper | 10-12 cm |
| 72 kg | Stomach Sleeper | 4-6 cm |
| 80 kg | Back Sleeper | 9-11 cm |
| 80 kg | Side Sleeper | 11-13 cm |
| 80 kg | Stomach Sleeper | 5-7 cm |

The processor 25 may be configured to identify the one or more suitable pillows from the plurality of pillows by comparing the target support height range or value and the target counter pressure range or value with the plurality of measured properties per pillow. Each plurality of measured properties per pillow comprises a plurality of pairs of pillow characteristics. Each of the pairs comprises a measured counter pressure and a corresponding value indicative of a support height. The control device 21 retrieves the pluralities of measured properties from a database 37.

The values indicative of the support height may be support height values or compression values, for example. A support height value may be determined from a compression value by subtracting the compression value from the pillow height.

In the implementation of Fig. 4, the plurality of measured properties per pillow have been obtained by automatically controlling an artificial head to achieve the different support heights of a respective pillow of the plurality of pillows and measuring the corresponding counter pressure at each of the different support heights. A device 51 automatically controls the artificial head to achieve the different support heights and measuring the corresponding counter pressure at each of the different support heights. This device 51 is also referred to as "Pillow Indexer" in this specification.

This pillow indexer 51 stores the pluralities of measured properties in database 37. The system 20 may be a fully local system or may be distributed over multiple locations. As an example of the latter, the database 37 may be located in the cloud, e.g., in a secure Data Lake, and the control device 21 may be either local or also be located in the cloud. The pillow indexer 51 does not need to be connected to database 37 permanently. For example, it may be connected to database 37 whenever new pillows are/have been indexed. In an alternative implementation, the pillow indexer is not directly connected to database 37, but measured properties are received from the pillow indexer 51 and then stored in database 37 by one or more intermediate devices.

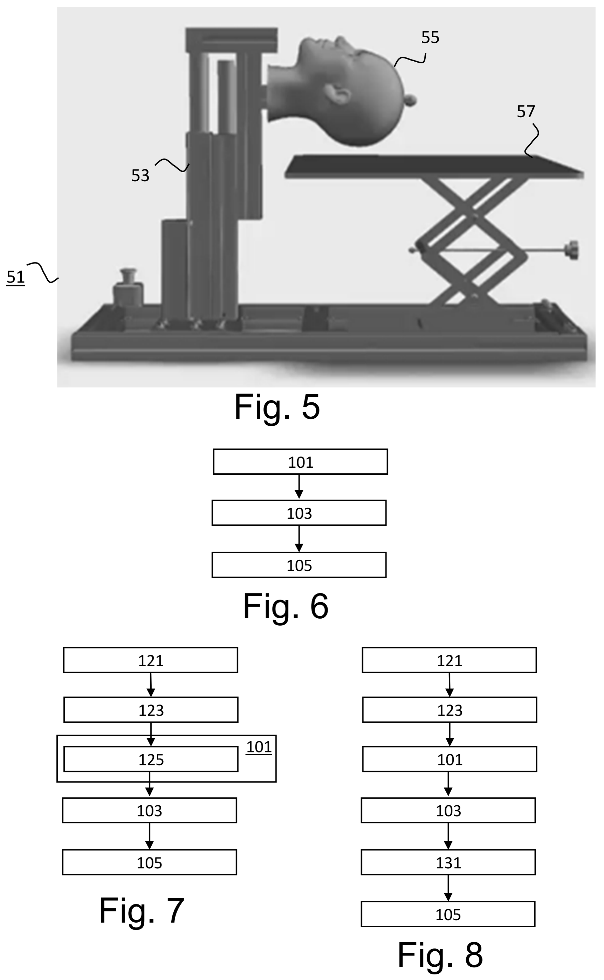

Fig. 5 shows a close-up view of the pillow indexer 51 of Fig. 4. The pillow indexer 51 comprises artificial head 55, a platform 57 on which the pillows-under-test are placed, and a motorized linear guide rail 53. The platform 57 comprises sensors configured to perform measurements indication of a respective force applied to the respective sensor. The platform 57 may comprise a 2x2 array of sensors like the head measuring component 33 of Fig. 3 but may alternatively comprise a different configuration/quantity of sensors. The sensors may be load cells, for example. The motorized linear guide rail 53 may be controllable via Bluetooth, for example.

The motorized linear guide rail 53 may be controlled to move in steps, e.g., in steps of 2 mm. At each step, the counter pressure may be measured. The measurements may be started as soon as the artificial head 55 touches the pillow-under-test. The displacement, i.e., distance, between the current position of the guide rail 53 and the position of the guide rail 53 when the artificial head 55 touched the pillow-under-test may be determined and stored as the compression associated with the measured counter pressure. The motorized linear guide rail 53 may be stopped as soon as a certain counter pressure is measured, e.g., 11 kilograms.

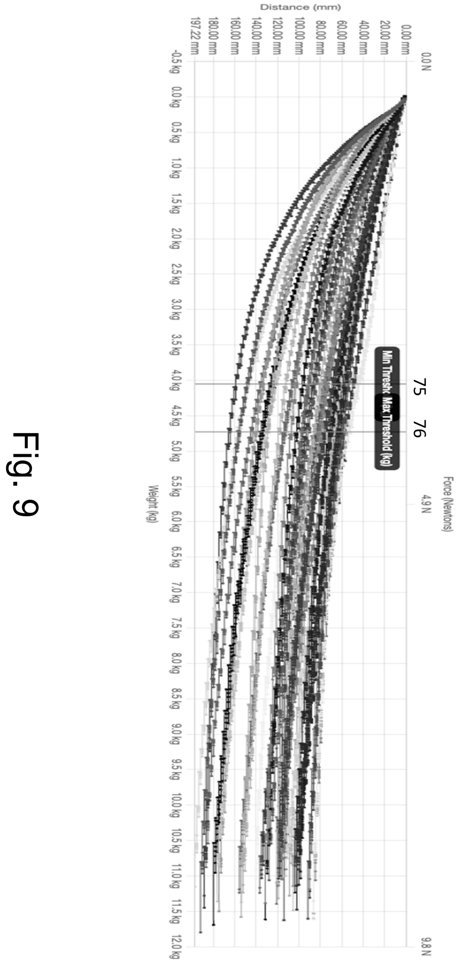

Figs. 9-11 show example measurement properties for multiple pillows. The measured counterpressure in kilograms is shown on the x-axis (also indicated in Newton). The compression (compressed height) in millimeters is shown on the y-axis.

In the example of Fig. 9, the processor 25 of Fig. 4 is configured to determine the one or more target properties for the target pillow by determining a target support height range and a target counter pressure range based on the first measurement results and the second measurement results. The target counter pressure range is between line 75 (minimum counter pressure) and line 76 (maximum counter pressure).

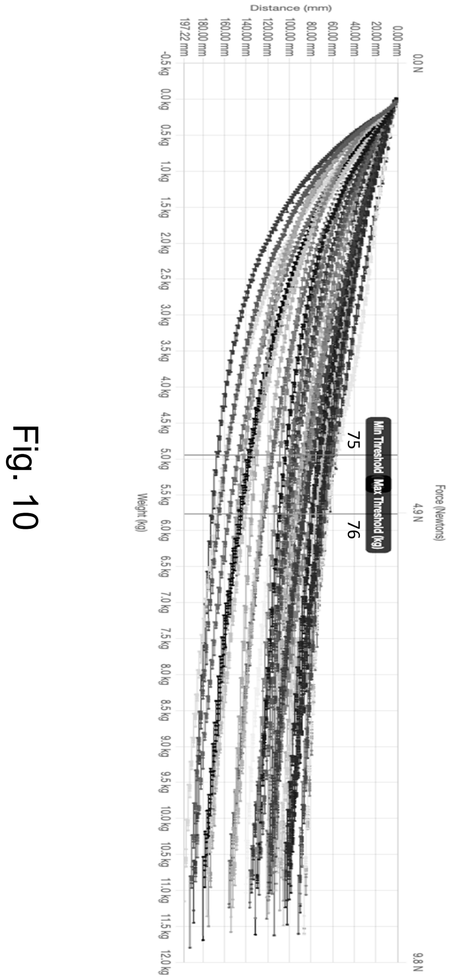

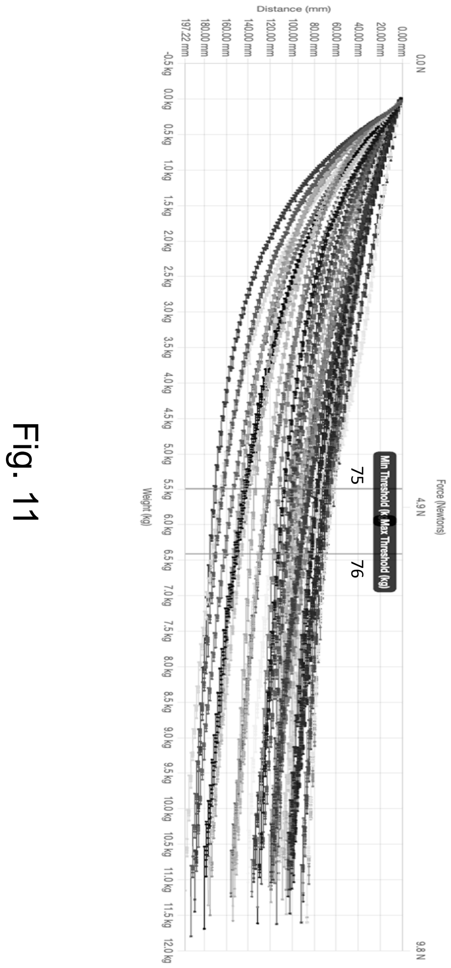

Fig. 9 shows the target counter pressure range for an average person of 59 kilograms with a balanced weight distribution. Fig. 10 shows the target counter pressure range for an average person of 72 kilograms with a balanced weight distribution. Fig. 11 shows the target counter pressure range for an average person of 80 kilograms with a balanced weight distribution.

As described above, the one or more target properties for the target pillow may be determined by determining a target support height range and a target counter pressure range based on the first measurement results and the second measurement results. It may be learned in several manners how to make this determination. An example of how to learn how to make this determination is provided below:
- A target support height value for a reference person is determined manually in the manner described before and using information from studies such as "Ergonomic Consideration in Pillow Height Determinants and Evaluation" by Jia-Xing Lei et al., published in Healthcare 2021, 9(10), 1333; https://doi.org/10.3390/healthcare9101333, and "P108 Effects of different pillow designs on promoting sleep quality and spinal alignment by reducing neck pain, waking symptoms, neck disability in adults: a systematic review and meta-analysis" by J. Pang, published in SLEEP Advances, Volume 2, Issue Supplement_1, October 2021, Page A56, https://doi.org/10.1093/sleepadvances/zpab014.151.
- The artificial head 55 (shape, dimensions, weight) is modelled based on a reference person. Optionally, the artificial head 55 is modelled based on a certain test subject, e.g., an average-type person, and the customer indexer is then used on this certain test subject. Optionally, the target support height value is verified and/or finetuned based on the measurements of the customer indexer on this test subject.
- A deviation from the target support height value is manually determined for persons with other body types. Optionally the customer indexer is used on (e.g., ten) additional test subjects with different body types and a deviation from the target support height value is manually determined for each additional person based on a deviation of the measurements performed on the additional person from the measurements performed on the reference person.
- The relation between deviation in measurements and deviation in support height value is then modelled by a mathematical function (e.g., by using machine learning) based on the deviations determined for the additional test subjects.
- In operation, when a customer uses the customer indexer, the deviation of the measurements of this customer from the measurements of the reference person are determined and a target deviation from the default support height value (determined for the reference person) is determined for this customer based on the mathematical function and this deviation in measurements. The measured force/pressure on the head measuring component 33 may be used as target counter support value, optionally after a certain adjustment has been made. An adjustment may be made to take into account the height of the head measuring component 33 (e.g. 3 centimeters).
- A high pillow with low firmness and a low pillow with high firmness may both be able to provide a certain target support height value or range.

The motorized linear guide rail 53 may also be controlled to rotate the artificial head 55. A first subset of the plurality of measured properties per pillow may have been obtained by automatically controlling the artificial head 55 with a back of the artificial head 55 (this assumes that the person is lying on their back) and a second subset of the plurality of measured properties per pillow may have been obtained by automatically controlling the artificial head 55 with a side of the artificial head 55 (this assumes that the person is lying on their side).

In the implementation of Fig. 4, the customer indexer also comprises a user terminal 35 configured to provide questions to a user and receive answers to the questions from the user. The user terminal 35 may have a touchscreen display, for example. The user terminal 35 may be a fixed terminal, e.g., located near the customer indexer, or a user device, e.g., a mobile phone. The questions may ask the user about one or more of: a firmness of a mattrass of the user, a favorite sleeping position of the user (e.g., side, back, or stomach), pain experienced by the user when lying in bed, comfortability of the user in terms of temperature when lying in bed, and materials to be excluded. These answers are provided to the control device 21.

The processor 25 of the control unit is configured is further configured to determine the one or more target properties for the target pillow further based on at least a subset of the answers and/or to select the plurality of pillows from a collection of pillows based on at least a subset of the answers. Specifications (e.g., material, warm or cool) of the collection of pillows may be obtained from a product information database and used to select the plurality of pillows from the collection of pillows based on at least a subset of the answers. This product database may be part of, or combined with, database 37, or may be a separate database.

Optionally, the customer indexer may comprise an adjustable bed base and a controller for adjusting the adjustable bed base based on a user's answer to a question about the firmness of the mattrass of the user (not shown in Fig. 4). This answer may then additionally be used to determine the one or more target properties for the target pillow. Information on the identified one or more pillows may be displayed on the user terminal 35. Alternatively, information on the identified one or more pillows may be sent to the customer by e-mail or printed.

In the implementation of Fig. 4, the body measuring component 31 comprises four sensors. In an alternative implementation, the body measuring component 31 comprises less or more than four sensors. In the implementation of Fig. 4, the head measuring component 33 comprises four sensors. In an alternative implementation, the head measuring component 33 comprises less or more than four sensors.

In the implementation of Fig. 4, the control device 21 has only one processor. In an alternative implementation, the control device 21 has multiple processors. The body and head measuring components 31 and 33 may each comprise a transmitter for transmitting measurements to the control device 21 or the customer indexer may comprise a component which receives the measurements from the body and head measuring components 31 and 33 and provides these to the control device 21. Optionally, this component may be the user terminal 35.

The receiver 23 and the transmitter 24 may be combined in a transceiver. The receiver 23 and the transmitter 24 may receive and transmit wireless signals (e.g., Bluetooth and/or Wi-Fi) and/or may receive and transmit wired signals (e.g., Ethernet). The memory 27 may store firmware and/or an operating system and may temporarily store data received from the customer indexer, the user terminal 35, and the database 37, for example.

A first implementation of the method of identifying one or more suitable pillows for a person based on measurements is shown in Fig. 6. The method may be performed by the control device 21 of Fig. 4, for example. A step 101 comprises obtaining first measurement results and second measurement results. The first measurement results comprise first measurements or are derived from the first measurements. The second measurement results comprise second measurements or are derived from the second measurements.

The first measurements are indicative of a respective force applied to a respective sensor of at least one first sensor and at least one second sensor. The at least one first sensor is positioned at at least one position of a pillow which corresponds to a top part of the person's head. The at least one second sensor is positioned at at least one position of the pillow which corresponds to a bottom part of the person's head.

The second measurements are indicative of a respective force applied to a respective sensor of at least one third sensor and at least one fourth sensor. The at least one third sensor is positioned at at least one position of a bed which corresponds to a top part of the person's body. The at least one fourth sensor is positioned at at least one position of the bed which corresponds to a bottom part of the person's body.

A step 103 comprises determining one or more target properties for a target pillow based on the first measurement results and the second measurement results obtained in step 101. A step 105 comprises identifying the one or more suitable pillows from a plurality of pillows by comparing the one or more target properties determined in step 103 with a plurality of measured properties per pillow of the plurality of pillows.

A second implementation of the method of identifying one or more suitable pillows for a person based on measurements is shown in Fig. 7. The method may be performed by the control device 21 of Fig. 4, for example. The implementation of Fig. 7 is an extension of the implementation of Fig. 6. In the implementation of Fig. 7, steps 121 and 123 are performed before step 101 of Fig. 6 and step 101 of Fig. 6 has been implemented by a step 125.

Step 121 comprises providing questions to a user. The questions may ask the user about one or more of: a firmness of a mattrass of the user, a favorite sleeping position of the user, pain experienced by the user when lying in bed, comfortability of the user in terms of temperature when lying in bed, and materials to be excluded. Step 123 comprises receiving answers to the questions from the user.

Step 125 comprises determining the one or more target properties for the target pillow further based on at least a subset of the answers received in step 123. This allows further customer information (e.g., preferences) to be taken into account that is not (sufficiently) reflected by the measurements. This may be used to ensure that the identified one or more pillows better match the customer's preferences. The implementations of Figs. 7 and 8 may be combined.

A third implementation of the method of identifying one or more suitable pillows for a person based on measurements is shown in Fig. 8. The method may be performed by the control device 21 of Fig. 4, for example. The implementation of Fig. 8 is an extension of the implementation of Fig. 6. In the implementation of Fig. 8, steps 121 and 123 are performed before step 101 of Fig. 6 and a step 131 is performed before step 105 of Fig. 6.

Step 121 comprises providing questions to a user. The questions may ask the user about one or more of: a firmness of a mattrass of the user, a favorite sleeping position of the user, pain experienced by the user when lying in bed, comfortability of the user in terms of temperature when lying in bed, and materials to be excluded. Step 123 comprises receiving answers to the questions from the user.

Step 131 comprises selecting a plurality of pillows from a collection of pillows based on at least a subset of the answers received in step 123. Step 105 comprises identifying the one or more suitable pillows from the plurality of pillows selected in step 131 by comparing the one or more target properties determined in step 103 with a plurality of measured properties per pillow of the plurality of pillows. This allows further customer information (e.g., preferences) to be taken into account that is not (sufficiently) reflected by the measurements. This may be used to ensure that the identified one or more pillows better match the customer's preferences. The implementations of Figs. 7 and 8 may be combined.

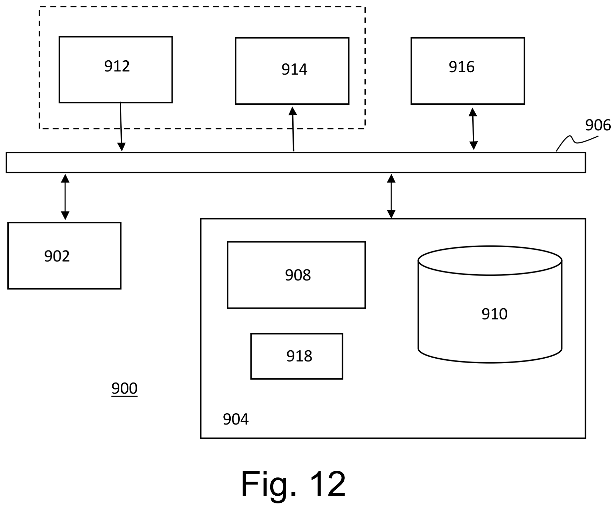

Fig. 12 depicts a block diagram illustrating an exemplary data processing system that may perform the method as described with reference to the flow charts.

As shown in Fig. 12, the data processing system 900 may include at least one processor 902 coupled to memory elements 904 through a system bus 906. As such, the data processing system may store program code within memory elements 904. Further, the processor 902 may execute the program code accessed from the memory elements 904 via a system bus 906. In one aspect, the data processing system may be implemented as a computer that is suitable for storing and/or executing program code. It should be appreciated, however, that the system 900 may be implemented in the form of any system including a processor and a memory that is capable of performing the functions described within this specification. The data processing system may be an Internet/cloud server, for example.

The memory elements 904 may include one or more physical memory devices such as, for example, local memory 908 and one or more bulk storage devices 910. The local memory may refer to random access memory or other non-persistent memory device(s) generally used during actual execution of the program code. A bulk storage device may be implemented as a hard drive or other persistent data storage device. The processing system 900 may also include one or more cache memories (not shown) that provide temporary storage of at least some program code in order to reduce the quantity of times program code must be retrieved from the bulk storage device 910 during execution. The processing system 900 may also be able to use memory elements of another processing system, e.g. if the processing system 900 is part of a cloud-computing platform.

Input/output (I/O) devices depicted as an input device 912 and an output device 914 optionally can be coupled to the data processing system. Examples of input devices may include, but are not limited to, a keyboard, a pointing device such as a mouse, a microphone (e.g. for voice and/or speech recognition), or the like. Examples of output devices may include, but are not limited to, a monitor or a display, speakers, or the like. Input and/or output devices may be coupled to the data processing system either directly or through intervening I/O controller.

The input and the output devices may be implemented as a combined input/output device (illustrated in Fig. 12 with a dashed line surrounding the input device 912 and the output device 914). An example of such a combined device is a touch sensitive display, also sometimes referred to as a "touch screen display" or simply "touch screen". In such an implementation, input to the device may be provided by a movement of a physical object, such as e.g. a stylus or a finger of a user, on or near the touch screen display.

A network adapter 916 may also be coupled to the data processing system to enable it to become coupled to other systems, computer systems, remote network devices, and/or remote storage devices through intervening private or public networks. The network adapter may comprise a data receiver for receiving data that is transmitted by the systems, devices and/or networks to the data processing system 900, and a data transmitter for transmitting data from the data processing system 900 to the systems, devices and/or networks. Modems, cable modems, and Ethernet cards are examples of different types of network adapter that may be used with the data processing system 900.

As pictured in Fig. 12, the memory elements 904 may store an application 918. The application 918 may be stored in the local memory 908, the one or more bulk storage devices 910, or separate from the local memory and the bulk storage devices. It should be appreciated that the data processing system 900 may further execute an operating system (not shown in Fig. 12) that can facilitate execution of the application 918. The application 918, being implemented in the form of executable program code, can be executed by the data processing system 900, e.g., by the processor 902. Responsive to executing the application, the data processing system 900 may be configured to perform one or more operations or method steps described herein.

The invention may be implemented as a program product for use with a computer system, where the program(s) of the program product define functions. The program(s) may be contained on a variety of non-transitory computer-readable storage media, where, as used herein, the expression "non-transitory computer readable storage media" comprises all computer-readable media, with the sole exception being a transitory, propagating signal. The program(s) may also be contained on a variety of transitory computer-readable storage media. Illustrative computer-readable storage media include, but are not limited to: (i) non-writable storage media (e.g., read-only memory devices within a computer such as CD-ROM disks readable by a CD-ROM drive, ROM chips or any type of solid-state non-volatile semiconductor memory) on which information is permanently stored; and (ii) writable storage media (e.g., flash memory, floppy disks within a diskette drive or hard-disk drive or any type of solid-state random-access semiconductor memory) on which alterable information is stored. The computer program may be run on the processor 902 described herein.

The terminology used herein is for the purpose of describing particular implementations only and is not intended to be limiting of the invention. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The detailed description has been presented for purposes of illustration, but is not intended to be exhaustive or limited to the implementations in the form disclosed. Many modifications and variations will be apparent to those of ordinary skill in the art without departing from the scope of the present invention.

## Claims

1. A system (20) for performing measurements in relation to a person, the measurements enabling one or more suitable pillows to be identified for the person, the system (20) comprising:
- a head measuring component (33) in the form of a pillow, the head measuring component (33) comprising at least one head sensor (51-54), the at least one head sensor (51-54) being configured to perform first measurements indicative of a respective force applied to the respective sensor, and
- a body measuring component (31) in the form of a bed, the body measuring component (31) comprising at least one third sensor (43,44) at at least one position of the bed which corresponds to a top part of the person's body and at least one fourth sensor (41,42) at at least one position of the bed which corresponds to a bottom part of the person's body, the at least one third sensor (43,44) and the at least one fourth sensor (41,42) being configured to perform second measurements indicative of a respective force applied to the respective sensor.

2. A system (20) as claimed in claim 1, wherein the body measuring component (31) comprises at least two third sensors (43,44) at first and second positions of the bed which correspond to a top part of the person's body and at least two fourth sensors (41,42) at third and fourth positions of the bed which correspond to a bottom part of the person's body, the first and third positions of the bed corresponding to a left part of the person's body and the second and fourth positions of the bed corresponding to a right part of the person's body.

3. A system (20) as claimed in claim 1 or 2, wherein the at least one head sensor (51-54) comprises at least one first sensor (53,54) at at least one position of the pillow which corresponds to a top part of the person's head and at least one second sensor (51,52) at at least one position of the pillow which corresponds to a bottom part of the person's head.

4. A system (20) as claimed in any one of the preceding claims, wherein the bed comprises one or more ground supports (61-63) which allow the bed to stand on the ground and the at least one third sensor (43,44) and the at least one fourth sensor (41,42) are mounted below the one or more ground supports (61-63).

5. A system (20) as claimed in any one of the preceding claims, further comprising at least one processor (25) configured to:
- obtain first measurement results and second measurement results, the first measurement results comprising the first measurements or being derived from the first measurements, the second measurement results comprising the second measurements or being derived from the second measurements,
- determine one or more target properties for a target pillow based on the first measurement results and the second measurement results, and
- identify the one or more suitable pillows from a plurality of pillows by comparing the one or more target properties with a plurality of measured properties per pillow of the plurality of pillows.

6. A system (20) as claimed in claim 5, wherein the at least one processor (25) is configured to:
- determine the one or more target properties for the target pillow by determining a target support height range or value and a target counter pressure range or value based on the first measurement results and the second measurement results, and
- identify the one or more suitable pillows from the plurality of pillows by comparing the target support height range or value and the target counter pressure range or value with the plurality of measured properties per pillow, the plurality of measured properties per pillow comprising a plurality of pairs of pillow characteristics per pillow, each of the pairs comprising a measured counter pressure and a corresponding value indicative of a support height.

7. A system (20) as claimed in claim 6, wherein the plurality of measured properties per pillow have been obtained by automatically controlling an artificial head (55) to achieve the different support height of a respective pillow of the plurality of pillows and measuring the corresponding counter pressure at each of the different support heights.

8. A system (20) as claimed in claim 7, wherein a first subset of the plurality of measured properties per pillow have been obtained by automatically controlling the artificial head (55) with a back of the artificial head (55) and a second subset of the plurality of measured properties per pillow have been obtained by automatically controlling the artificial head with a side of the artificial head (55).

9. A system (20) as claimed in claim 7 or 8, further comprising a device for automatically controlling the artificial head (55) to achieve the different support heights and measuring the corresponding counter pressure at each of the different support heights.

10. A system (20) as claimed in any one of claims 5 to 9, further comprising a user terminal (35) configured to provide questions to a user and receive answers to the questions from the user, and wherein the at least one processor (25) is further configured to determine the one or more target properties for the target pillow further based on at least a subset of the answers and/or to select the plurality of pillows from a collection of pillows based on at least a subset of the answers.

11. A system (20) as claimed in claim 10, wherein the questions ask the user about one or more of: a firmness of a mattrass of the user, a favorite sleeping position of the user, pain experienced by the user when lying in bed, comfortability of the user in terms of temperature when lying in bed, and materials to be excluded.

12. A system (20) as claimed in claim 11, wherein one of the questions asks the user about the firmness of the mattrass of the user and the system (20) further comprises an adjustable bed base and a controller for adjusting the adjustable bed base based on the user's answer to the question about the firmness of the mattrass of the user.

13. A system (20) as claimed in any one of claims 5 to 12 when dependent on claim 2, wherein the second measurement results comprise at least one average top measurement and at least one average bottom measurement, the average top measurement being an average of measurements performed by the least two third sensors (43,44) at the first and second positions of the bed and the average bottom measurement being an average of measurements performed by the least two fourth sensors (41,42) at the third and fourth positions of the bed,
and wherein the at least one processor (25) is configured to determine the one or more target properties for the target pillow based on the first measurement results, the average top measurement, and the average bottom measurement.

14. A method of identifying one or more suitable pillows for a person based on measurements, the method comprising:
- obtaining (101) first measurement results and second measurement results, the first measurement results comprising first measurements or being derived from the first measurements, the second measurement results comprising second measurements or being derived from the second measurements, the first measurements being indicative of a respective force applied to a respective sensor of at least one head sensor, the second measurements being indicative of a respective force applied to a respective sensor of at least one third sensor and at least one fourth sensor, the at least one third sensor being positioned at at least one position of a bed which corresponds to a top part of the person's body and the at least one fourth sensor being positioned at at least one position of the bed which corresponds to a bottom part of the person's body,
- determining (103) one or more target properties for a target pillow based on the first measurement results and the second measurement results, and
- identifying (105) the one or more suitable pillows from a plurality of pillows by comparing the one or more target properties with a plurality of measured properties per pillow of the plurality of pillows.

15. A computer program product for a computing device, the computer program product comprising computer program code to perform the method of claim 14 when the computer program product is run on a processing unit of the computing device.
